# EUROPÄISCHE PATENTSCHRIFT

(11) **EP 1 052 252 B1**
(45) Veröffentlichungstag und Bekanntmachung des Hinweises auf die Patenterteilung: **06.08.2003**
(21) Anmeldenummer: 00102127.8
(22) Anmeldetag: 04.02.2000
(51) Int. Cl.: C07D 213/38, C07D 213/85, C07D 213/61, C07D 213/26, C07D 213/82, C07D 213/73, C07D 213/64, C07D 213/65, C07D 213/68, C07D 213/84, C07D 239/26, A61K 7/13

(54) **Diaminobenzol-Derivate und diese Verbindungen enthaltende Färbemittel**
Diaminobenzene derivatives and dyeing preparations containing them
Derivés du diamino-benzène et compositions pour la teinture les contenant

(30) Priorität: 14.05.1999 DE 19922392
(43) Veröffentlichungstag der Anmeldung: 15.11.2000
(73) Patentinhaber: Wella Aktiengesellschaft, 64274 Darmstadt (DE)
(72) Erfinder: Chassot, Laurent, Dr., 1724 Praroman (CH); Descloux, Laurence, 1756 Lovens (CH)

(56) Entgegenhaltungen:
- EP-A- 0 740 931
- EP-A- 0 943 614
- EP-A- 0 963 982
- DE-A- 2 518 393
- DE-A- 19 822 041
- US-A- 3 647 351
- US-A- 4 994 087

## Beschreibung

Die Erfindung betrifft neue p-Diaminobenzol-Derivate sowie diese Verbindungen enthaltende Mittel zum Färben von Keratinfasern.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol, 5-Amino-2-methylphenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Aus der US-PS 3 647 351 sind Oxidationshaarfärbemittel bekannt, welche spezielle Pyridinderivate, zum Beispiel das 6-Amino-pyridyl-amino(2)-1,4-diaminobenzol, als Farbstoffvorstufen enthalten. Die mit diesen Verbindungen erhaltenen Färbungen sind jedoch im Hinblick auf ihre Farbintensität nicht völlig befriedigend. Aus der DE-OS 25 18 393 sind Haarfärbemittel bekannt, welche als Entwicklersubstanz spezielle Tetraaminodiphenyle, Diamino-dihydroxy-diphenyle oder Tetraaminodiphenylether und Diamino-dihydroxy-diphenylether, wie zum Beispiel das 2,2',5,5'-Tetraaminodiphenyl, enthalten. Die Färbungen mit diesen Färbemitteln sind jedoch ebenfalls nicht befriedigend, da die Erzielung einer ausreichenden Farbsättigung sowie die Erzielung von reinen Farbtönen -insbesondere bei Verwendung von Blaukupplern- nicht immer möglich ist.

Da mit den derzeit bekannten und eingesetzten Färbemitteln es nicht möglich ist, die an Färbemittel gestellten Anforderungen in jeder Hinsicht zu erfüllen, bestand weiterhin ein Bedürfnis nach neuen Entwicklersubstanzen, welche die vorgenannten Anforderung in besonderem Masse erfüllen.

Hierzu wurde nun überraschenderweise gefunden, daß neue, in 2-Stellung mit einem Pyridyl- oder Pyrimidylrest substituierte p-Diaminobenzol-Derivate die an Entwicklersubstanzen gestellten Anforderungen in besonders hohem Masse erfüllen. So werden unter Verwendung dieser Entwicklersubstanzen mit den meisten bekannten Kupplersubstanzen farbstarke Farbnuancen erhalten, die außerordentlich lichtecht und waschecht sind.

Gegenstand der vorliegenden Erfindung sind daher p-Diaminobenzol-Derivate, die ausgewählt sind aus der Gruppe bestehend aus 2,5-Diamino-1-(2-pyridyl)benzol; 2,5-Diamino-1-(3-pyridyl)benzol; 2,5-Diamino-1-(4-pyridyl)benzol; 2,5-Diamino-4-methoxy-1-(2-pyridyl)-benzol; 2,5-Diamino-4-methoxy-1-(3-pyridyl)benzol; 2,5-Diamino-4-methoxy-1-(4-pyridyl)benzol; 2,5-Diamino-4-methyl-1-(2-pyridyl)benzol; 2,5-Diamino-4-methyl-1-(3-pyridyl)benzol; 2,5-Diamino-4-methyl-1-(4-pyridyl)benzol; 2,5-Diamino-1-(3-amino-2-pyridyl)benzol; 2,5-Diamino-1-(3-chlor-2-pyridyl)benzol; 2,5-Diamino-1-(3-fluor-2-pyridyl)benzol; 2,5-Diamino-1-(3-hydroxy-2-pyridyl)benzol; 2,5-Diamino-1-(3-methyl-2-pyridyl)benzol; 2,5-Diamino-1-(3-trifluormethyl-2-pyridyl)benzol; 2,5-Diamino-1-(4-amino-2-pyridyl)benzol; 2,5-Diamino-1-(4-chlor-2-pyridyl)benzol; 2,5-Diamino-1-(4-fluor-2-pyridyl)benzol; 2,5-Diamino-1-(4-hydroxy-2-pyridyl)benzol; 2,5-Diamino-1-(4-methyl-2-pyridyl)benzol; 2,5-Diamino-1-(4-trifluormethyl-2-pyridyl)benzol; 2,5-Diamino-1-(5-amino-2-pyridyl)benzol; 2,5-Diamino-1-(5-chlor-2-pyridyl)benzol; 2,5-Diamino-1-(5-fluor-2-pyridyl)benzol; 2,5-Diamino-1-(5-hydroxy-2-pyridyl)benzol; 2,5-Diamino-1-(5-methyl-2-pyridyl)benzol; 2,5-Diamino-1-(5-trifluormethyl-2-pyridyl)benzol; 2,5-Diamino-1-(6-amino-2-pyridyl)benzol; 2,5-Diamino-1-(6-chlor-2-pyridyl)benzol; 2,5-Diamino-1-(6-fluor-2-pyridyl)benzol; 2,5-Diamino-1-(6-hydroxy-2-pyridyl)benzol; 2,5-Diamino-1-(6-methyl-2-pyridyl)benzol; 2,5-Diamino-1-(6-trifluormethyl-2-pyridyl)benzol; 2,5-Diamino-1-(2-amino-3-pyridyl)benzol; 2,5-Diamino-1-(2-chlor-3-pyridyl)benzol; 2,5-Diamino-1-(2-fluor-3-pyridyl)benzol; 2,5-Diamino-1-(2-hydroxy-3-pyridyl)benzol; 2,5-Diamino-1-(2-methyl-3-pyridyl)benzol; 2,5-Diamino-1-(2-trifluormethyl-3-pyridyl)benzol; 2,5-Diamino-1-(4-amino-3-pyridyl)benzol; 2,5-Diamino-1-(4-chlor-3-pyridyl)benzol; 2,5-Diamino-1-(4-fluor-3-pyridyl)benzol; 2,5-Diamino-1-(4-hydroxy-3-pyridyl)benzol; 2,5-Diamino-1-(4-methyl-3-pyridyl)benzol; 2,5-Diamino-1-(4-trifluormethyl-3-pyridyl)benzol; 2,5-Diamino-1-(5-amino-3-pyridyl)benzol; 2,5-Diamino-1-(5-chlor-3-pyridyl)benzol; 2,5-Diamino-1-(5-fluor-3-pyridyl)benzol; 2,5-Diamino-1-(5-hydroxy-3-pyridyl)benzol; 2,5-Diamino-1-(5-methyl-3-pyridyl)benzol; 2,5-Diamino-1-(5-trifluormethyl-3-pyridyl)benzol; 2,5-Diamino-1-(6-amino-3-pyridyl)benzol; 2,5-Diamino-1-(6-chlor-3-pyridyl)benzol; 2,5-Diamino-1-(6-fluor-3-pyridyl)benzol; 2,5-Diamino-1-(6-hydroxy-3-pyridyl)benzol; 2,5-Diamino-1-(6-methyl-3-pyridyl)benzol; 2,5-Diamino-1-(6-trifluormethyl-3-pyridyl)benzol; 2,5-Diamino-1-(2-amino-4-pyridyl)benzol; 2,5-Diamino-1-(2-chlor-4-pyridyl)benzol; 2,5-Diamino-1-(2-fluor-4-pyridyl)benzol; 2,5-Diamino-1-(2-hydroxy-4-pyridyl)benzol; 2,5-Diamino-1-(2-methyl-4-pyridyl)benzol; 2,5-Diamino-1-(2-trifluormethyl-4-pyridyl)benzol; 2,5-Diamino-1-(3-amino-4-pyridyl)benzol; 2,5-Diamino-1-(3-chlor-4-pyridyl)benzol; 2,5-Diamino-1-(3-fluor-4-pyridyl)benzol; 2,5-Diamino-1-(3-hydroxy-4-pyridyl)benzol; 2,5-Diamino-1-(3-methyl-4-pyridyl)benzol; 2,5-Diamino-1-(3-trifluormethyl-4-pyridyl)benzol; 2,5-Diamino-1-(5-amino-4-pyridyl)benzol; 2,5-Diamino-1-(5-chlor-4-pyridyl)benzol; 2,5-Diamino-1-(5-fluor-4-pyridyl)benzol; 2,5-Diamino-1-(5-hydroxy-4-pyridyl)benzol; 2,5-Diamino-1-(5-methyl-4-pyridyl)benzol; 2,5-Diamino-1-(5-trifluormethyl-4-pyridyl)benzol; 2,5-Diamino-1-(6-amino-4-pyridyl)benzol; 2,5-Diamino-1-(6-chlor-4-pyridyl)benzol; 2,5-Diamino-1-(6-fluor-4-pyridyl)benzol; 2,5-Diamino-1-(6-hydroxy-4-pyridyl)benzol; 2,5-Diamino-1-(6-methyl-4-pyridyl)benzol; 2,5-Diamino-1-(6-trifluormethyl-4-pyridyl)benzol; 2,5-Diamino-1-(2-pyrimidyl)benzol; 2,5-Diamino-1-(4-pyrimidyl)benzol; 2,5-Diamino-1-(5-pyrimidyl)benzol; 2,5-Diamino-1-(6-pyrimidyl)benzol; 2-Hydroxyethylamino-5-amino-1-(2-pyridyl)benzol; 2-Hydroxyethylamino-5-amino-1-(2-pyridyl)benzol; 2-Hydroxyethylamino-5-amino-1-(3-pyridyl)benzol; 2-Hydroxyethylamino-5-amino-1-(4-pyridyl)-benzol; 2-Bis(hydroxyethyl)amino-5-amino-1-(2-pyridyl)benzol; 2-Bis-(hydroxyethyl)amino-5-amino-1-(3-pyridyl)benzol; 2-Bis(hydroxyethyl)-amino-5-amino-1-(4-pyridyl)benzol; 2,5-Diamino-1-(3-cyan-2-pyridyl)-benzol; 2,5-Diamino-1-(3-nitro-2-pyridyl)benzol; 2,5-Diamino-1-(4-cyan-2-pyridyl)benzol; 2,5-Diamino-1-(4-nitro-2-pyridyl)benzol; 2,5-Diamino-1-(5-cyan-2-pyridyl)benzol; 2,5-Diamino-1-(5-nitro-2-pyridyl)benzol; 2,5-Diamino-1-(6-cyan-2-pyridyl)benzol; 2,5-Diamino-1-(6-nitro-2-pyridyl)benzol; 2,5-Diamino-1-(2-cyan-4-pyridyl)benzol; 2,5-Diamino-1-(2-nitro-4-pyridyl)benzol; 2,5-Diamino-1-(3-cyan-4-pyridyl)benzol; 2,5-Diamino-1-(3-nitro-4-pyridyl)benzol; 2,5-Diamino-1-(5-cyan-4-pyridyl)benzol; 2,5-Diamino-1-(5-nitro-4-pyridyl)benzol; 2,5-Diamino-1-(6-cyan-4-pyridyl)benzol; 2,5-Diamino-1-(6-nitro-4-pyridyl)benzol; 2,5-Diamino-1-(2-cyan-3-pyridyl)benzol; 2,5-Diamino-1-(2-nitro-3-pyridyl)-benzol; 2,5-Diamino-1-(4-cyan-3-pyridyl)benzol; 2,5-Diamino-1-(4-nitro-3-pyridyl)benzol; 2,5-Diamino-1-(5-cyan-3-pyridyl)benzol; 2,5-Diamino-1-(5-nitro-3-pyridyl)benzol; 2,5-Diamino-1-(6-cyan-3-pyridyl)benzol und 2,5-Diamino-1-(6-nitro-3-pyridyl)benzol oder deren physiologisch verträglichen Salzen.

Besonders bevorzugte p-Diaminobenzol-Derivate sind hierbei 2,5-Diamino-1-(2-pyridyl)benzol; 2,5-Diamino-1-(3-pyridyl)benzol; 2,5-Diamino-1-(4-pyridyl)benzol; 2,5-Diamino-1-(2-pyrimidyl)benzol; 2,5-Diamino-1-(6-methyl-2-pyridyl)benzol; 2,5-Diamino-1-(4-methyl-2-pyridyl)benzol; 2,5-Diamino-1-(5-methyl-2-pyridyl)benzol und 2,5-Diamino-1-(3-methyl-2-pyridyl)benzol oder deren physiologisch verträgliche Salze.

Die erfindungsgemäßen Verbindungen können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Diaminobenzol-Derivate kann unter Verwendung von bekannten Syntheseverfahren erfolgen. Die Synthese der erfindungsgemäßen Verbindungen kann beispielsweise wie folgt durchgeführt werden:
Entweder **a)** durch eine Tetrakis(triphenylphospin)palladium katalysierte Kupplung eines substituierten Benzols der Formel (I) 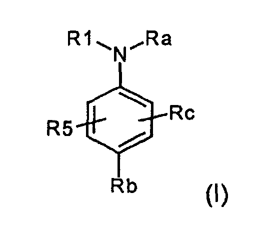 mit einer Pyridylverbindung oder Pyrimidylverbindung der Formel (lla) bzw. (llb)

   Pyridyl-Rd (IIa);

   Pyrimidyl-Rd (IIb)

   und anschließende Abspaltung der Schutzgruppe oder Abspaltung der Schutzgruppe und Reduktion der Nitrogruppe; oder
**b)** durch eine Tetrakis(triphenylphospin)palldium katalysierte Kupplung eines substituierten Benzols der Formel (III) 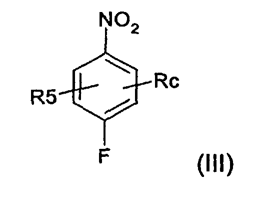 mit einer Pyridylverbindung oder Pyrimidylverbindung der Formel (lla) bzw. (IIb)

   Pyridyl-Rd (IIa);

   Pyrimidyl-Rd (IIb)

   und anschließende Substitution des so erhaltenen substituierten Benzols der Formel (IV) 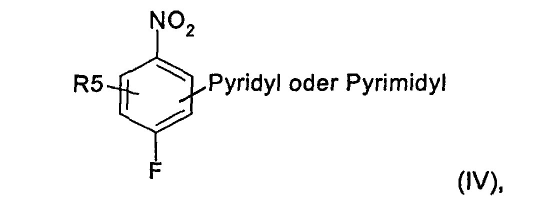 mit einem Amin der Formel HNR1R2 und anschließende Reduktion der Nitrogruppe.

Die in den Formeln (I) bis (IV) verwendeten Restgruppen haben hierbei die folgende Bedeutung:
**Ra** stellt eine Schutzgruppe dar, wie sie zum Beispiel in dem Kapitel "Protective Groups" in Organic Synthesis, Kapitel 7, Wiley Interscience, 1991 beschrieben wird;
**Rb** ist gleich NR1Ra oder NO₂;
**Rc** ist gleich Halogen und **Rd** ist gleich B(OH)₂ beziehungsweise
**Rc** ist gleich B(OH)₂ und **Rd** ist gleich Halogen;
**R1, R2** sind die entsprechenden Substituenten an der Aminogruppe (z.B. -CH2CH2OH);
**R5** ist der entsprechende Substituent am Benzolrest (z.B. -CH3 oder -OCH3).

Die erfindungsgemäßen Diaminobenzol-Derivate sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die erfindungsgemäßen Verbindungen weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein erfindungsgemäßes Diaminobenzol-Derivat enthalten.

Die erfindungsgemäßen Diaminobenzol-Derivate sind in dem erfindungsgemäßen Färbemittel in einer Gesamtmenge von etwa 0,005 bis 20 Gewichtsprozent enthalten, wobei eine Menge von etwa 0,01 bis 5,0 Gewichtsprozent und insbesondere von 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amlno-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-Indolindion in Betracht.

Obwohl die vorteilhaften Eigenschaften der erfindungsgemäßen Diaminobenzol-Derivate es nahelegen, diese als alleinige Entwicklersubstanz zu verwenden, ist es selbstverständlich auch möglich, diese Diaminobenzol-Derivate gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind (beispielsweise in einem Verhältnis von Kupplersubstanz zu Entwicklersubstanz von 1:2 bis 1:0,5).

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'hydroxyethyl)amino-nitrotoluol, 2-Amino-4,6-dinitrophenol, 2-Amino-5-(2'hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Vorzugsweise weist das erfindungsgemäße Färbemittel einen pH-Wert von 5 bis 11,5 auf, wobei ein pH-Wert von etwa 6,8 bis 10, 5 besonders bevorzugt ist. Die basische Einstellung erfolgt vorzugsweise mit Ammoniak, es können aber auch organische Amine, zum Beispiel Monoethanolamin und Triethanolamin, oder auch anorganische Basen wie Natriumhydroxid und Kaliumhydroxid verwendet werden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an den vorgenannten erfindungsgemäßen Diaminobenzol-Derivaten als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### I. Herstellungsbeispiele

### Beispiele 1: Synthese von 2,5 -Diamino-1-pyridylbenzol -Derivaten (Allgemeine Synthesevorschrift)

### A. Synthese von 2,5 -tert. -Butyloxycarbonylamino -brombenzol

15,65g (0,07 mol) Brom-p-phenylendiamin-Hydrochlorid und 32,7 g (0,15 mol) Di-tert.-butyl-dicarbonat werden in einer Mischung von 250 ml 2N Natriunhydroxide und 250 ml Trifluortoluol gelöst und auf 45 °C erwärmt. Die Reaktionmischung wird 3 Tage gerührt. Schrittweise werden noch insgesamt 30 g (0,14 mol) Di-tert.-butyl-dicarbonat zugegeben. Anschließend wird die organische Schicht abgetrennt und die wäßrige Phase noch zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylaminobrombenzol mit einem Schmelzpunkt von 130 °C erhalten.

### B. Synthese von 2,5 -Diamino-1-pyridylbenzol -Derivaten

3,3 g (0,01 mol) 2,5-tert.-Butyloxycarbonylamino-brombenzol aus Stufe **A** und 0,013 mol der entsprechenden Borsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonat-lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 40 ml Ethanol auf 50 °C erwärmt.
Anschließend werden zur Herstellung des Hydrochlorides 15 ml einer 2,9 molaren ethanolische Salzsäurelösung zugetropft. Der Niederschlag wird abfiltriert, zweimal mit 10 ml Ethanol gewaschen und sodann getrocknet. **a.** 2,5-Diamino-1-(3-pyridyl)-benzol -trihydrochlorid
Verwendetes Borsäure: 3-Pyridyl-borsäure
Ausbeute: 1,3 g (= 45 Prozent der Theorie)
Schmelzpunkt: 250 °Celsius (Zersetzung) (farblose Kristalle)

| CHN-Analyse: | | | |
|---|---|---|---|
| (C₁₁H₁₄N₃Cl₃) | %C | %H | %N |
| berechnet: | 44,85 | 4,79 | 14,26 |
| gefunden: | 45,56 | 4,26 | 14,09 |

### Beispiel 2: Synthese von 2,5-Diamino-1-pyridylbenzol-Derivaten und 2,5-Diamino-1-(2-pyrimidyl)benzol-Derivaten (Allgemeine Synthesevorschrift)

### A. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure wird durch Umsetzung von N,N'-Bis(tert-Butoxycarbonyl)-2,5-diamino-1-brombenzol mit tert-Butyllithium und Trimethylborate dargestellt. Die experimentelle Vorschrift dieser Herstellungsmethode wird von J. M. Tour und J. J. S: Lamba in J. Am. Chem. Soc.1994,116 Seite 11723 beschrieben.

### B. Synthese von 2,5-Diamino-1-pyridylbenzolen und 2,5-Diamino-1-(2-pyrimidyl)benzolen

0,035 g (0,0001 mol) 2,5-tert.-Butyloxycarbonylamino-1-phenylborsäure aus Stufe **A** und 0,00015 mol des entsprechenden Bromderivates werden unter Argon in 10 ml 1,2-Dimethoxyethan gelöst. Anschließend werden 0,005 g Tetrakis-(triphenylphosphin)-palladium (0,000005 mol) und 0,13 ml 2N Kaliumcarbonat-lösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 2,5-Diamino-1-(3-methyl-2-pyridyl)benzol-hydrochlorid

Verwendetes Bromderivat: 2-Brom-3-methylpyridin
Ausbeute: 0,025 g (81 % der Theorie)
Massenspektrum: MH⁺ 200 (100)

### c. 2,5-Diamino-1-(6-methyl-2-pyridyl)benzol-hydrochlorid

Verwendetes Bromderivat: 2-Brom-6-methylpyridin
Ausbeute: 0,025 g ( 81 % der Theorie)
Massenspektrum: MH⁺ 200 (100)

### d. 2,5-Diamino-1-(3-trifluormethyl-2-pyridyl)benzol-hydrochlorid

Verwendetes Bromderivat: 2-Brom-3-trifluormethylpyridin
Ausbeute: 0,025 g ( 69 % der Theorie)
Massenspektrum: MH⁺ 254 (100)

### e. 2,5-Diamino-1-(2-pyridyl)benzol-hydrochlorid

Verwendetes Bromderivat: 2-Brompyridin
Ausbeute: 0,025 g ( 85 % der Theorie)
Massenspektrum: MH⁺ 186 (100)

### f. 2,5-Diamino-1-(5-cyan-3-pyridyl)benzol-hydrochlorid

Verwendetes Bromderivat: 3-Brom-5-cyanpyridin
Ausbeute: 0,025 g ( 88 % der Theorie)
Massenspektrum: MH⁺ s38 (100)

### g. 2,5-Diamino-1-(5-methyl-2-pyridyl)benzol-hydrochlorid

Verwendetes Bromderivat: 2-Brom-5-methylpyridin
Ausbeute: 0,025 g ( 81 % der Theorie)
Massenspektrum: MH⁺ 200 (100)

### h. 2,5-Diamino-1-(4-methyl-2-pyridyl)benzol-hydrochlorid

Verwendetes Bromderivat: 2-Brom-4-methylpyridin
Ausbeute: 0,025 g ( 81 % der Theorie)
Massenspektrum: MH⁺ 200 (100)

### i. 2,5-Diamino-1-(5-trifluormethyl-2-pyridyl)benzol-hydrochlorid

Verwendetes Bromderivat: 2-Brom-5-trifluormethylpyridin
Ausbeute: 0,025 g (69 % der Theorie)
Massenspektrum: MH⁺ 254 (100)

### j. 2,5-Diamino-1-(5-nitro-2-pyridyl)benzol-hydrochlorid

Verwendetes Bromderivat: 2-Brom-5-nitropyridin
Ausbeute: 0,025 g ( 74 % der Theorie)
Massenspektrum: MH⁺ 231(100)

### k. 2,5-Diamino-1-(2-pyrimidyl)benzol-hydrochlorid

Verwendetes Bromderivat: 2-Brompyrimidin
Ausbeute: 0,025 g ( 74 % der Theorie)
Massenspektrum: MH⁺ 231(100)

### II. Beispiele für Haarfärbemittel

### Beispiele 3 bis 6: Haarfärbemittel

Es werden Haarfärbelösungen der folgenden Zusammensetzung hergestellt:

| | |
|---|---|
| 0,00125 mol | Entwicklersubstanz gemäß Tabelle 1 |
| 0,00125 mol | Kupplersubstanz gemäß Tabelle 1 |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 1 zusammengefaßt.

**Tabelle 1**

| **Beispiel** | **Entwicklersubstanz der Formel (I)** | **Kupplersubstanz** | **erhaltene Färbung** |
|---|---|---|---|
| **3** | 2,5-Diamino-1-(3-pyridyl)benzol∗2HCl | 2-Amino-4-(2'-hydroxyethyl)-amino-anisolsulfat | dunkelblau |
| **4** | 2,5-Diamino-1-(3-pyridyl)benzol∗2HCl | Resorcin | dunkelblond |
| **5** | 2,5-Diamino-1-(3-pyridyl)benzol∗2HCl | m-Aminophenol | dunkelgrau |
| **6** | 2,5-Diamino-1-(3-pyridyl)benzol∗2HCl | 5-Amino-2-methylphenol | rot |

### Beispiele 7 bis 50: Haarfärbemittel

Es werden Haarfärbelösungen der folgenden Zusammensetzung hergestellt:

| | |
|---|---|
| 0,0000125 mol | Entwicklersubstanz gemäß Tabelle 2 |
| 0,0000125 mol | Kupplersubstanz gemäß Tabelle 2 |
| 0,01g | Kaliumoleat (8prozentige wäßrige Lösung) |
| 0,01g | Ammoniak (22prozentige wäßrige Lösung) |
| 0,01g | Ethanol |
| 0,003 g | Ascorbinsäure |
| ad 1,0 g | Wasser |

1 g der vorstehenden Färbelösung werden unmittelbar vor der Anwendung mit 1 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 2 zusammengefaßt.

**Tabelle 2:**

| **Beispiel** | **Entwicklersubstanz** | **Kupplersubstanz** | **erhaltene Färbung** |
|---|---|---|---|
| **7** | 2,5-Diamino-1-(6-methyl-2-pyridyl)benzol∗3HCl | 1,3-Diamino-4-(2-hydroxyethoxy)-benzol∗sulfat | dunkelblau |
| **8** | 2,5-Diamino-1-(6-methyl-2-pyridyl)benzol∗3HCl | 1-Naphtol | blau |
| **9** | 2,5-Diamino-1-(6-methyl-2-pyridyl)benzol∗3HCl | 5-Amino-2-methyl-phenol | rot |
| **10** | 2,5-Diamino-1-(6-methyl-2-pyridyl)benzol∗3HCl | Resorcin | dunkelblond |
| **15** | 2,5-Diamino-1-(3-methyl-2-pyridyl)benzol∗3HCl | 1,3-Diamino-4-(2-hydroxyethoxy)-benzol∗sulfat | dunkelblau |
| **16** | 2,5-Diamino-1-(3-methyl-2-pyridyl)benzol∗3HCl | 1-Naphtol | blau |
| **17** | 2,5-Diamino-1-(3-methyl-2-pyridyl)benzol∗3HCl | 5-Amino-2-methyl-phenol | rot |
| **18** | 2,5-Diamino-1-(3-methyl-2-pyridyl)benzol∗3HCl | Resorcin | dunkelblond |
| **19** | 2,5-Diamino-1-(3-trifluormethyl-2-pyridyl)benzol∗3HCl | 1,3-Diamino-4-(2-hydroxyethoxy)-benzol∗sulfat | dunkelblau |
| **20** | 2,5-Diamino-1-(3-trifluormethyl-2-pyridyl)benzol∗3HCl | 1-Naphtol | blau |
| **21** | 2,5-Diamino-1-(3-trifluormethyl-2-pyridyl)benzol∗3HCl | 5-Amino-2-methyl-phenol | rot |
| **22** | 2,5-Diamino-1-(3-trifluormethyl-2-pyridyl)benzol∗3HCl | Resorcin | dunkelblond |
| **23** | 2,5-Diamino-1-(2-pyridyl)benzol∗3HCl | 1,3-Diamino-4-(2-hydroxyethoxy)-benzol∗sulfat | dunkelblau |
| **24** | 2,5-Diamino-1-(2-pyridyl)benzol∗3HCl | 1-Naphtol | blau |
| **25** | 2,5-Diamino-1-(2-pyridyl)benzol∗3HCl | 5-Amino-2-methyl-phenol | rot |
| **26** | 2,5-Diamino-1-(2-pyridyl)benzol∗3HCl | Resorcin | dunkelblond |
| **27** | 2,5-Diamino-1-(5-cyan-3-pyridyl)benzol∗3HCI | 1,3-Diamino-4-(2-hydroxyethoxy)-benzol∗sulfat | dunkelblau |
| **28** | 2,5-Diamino-1-(5-cyan-3-pyridyl)benzol∗3HCl | 1-Naphtol | blau |
| **29** | 2,5-Diamino-1-(5-cyan-3-pyridyl)benzol∗3HCl | 5-Amino-2-methyl-phenol | rot |
| **30** | 2,5-Diamino-1-(5-cyan-3-pyridyl)benzol∗3HCl | Resorcin | dunkelblond |
| **31** | 2,5-Diamino-1-(5-methyl-2-pyridyl)benzol∗3HCl | 1,3-Diamino-4-(2-hydroxyethoxy)-benzol∗sulfat | dunkelblau |
| **32** | 2,5-Diamino-1-(5-methyl-2-pyridyl)benzol∗3HCI | 1-Naphtol | blau |
| **33** | 2,5-Diamino-1-(5-methyl-2-pyridyl)benzol∗3HCI | 5-Amino-2-methyl-phenol | rot |
| **34** | 2,5-Diamino-1-(5-methyl-2-pyridyl)benzol∗3HCl | Resorcin | dunkelblond |
| **35** | 2,5-Diamino-1-(4-methyl-2-pyridyl)benzol∗3HCl | 1,3-Diamino-4-(2-hydroxyethoxy)-benzol∗sulfat | dunkelblau |
| **36** | 2,5-Diamino-1-(4-methyl-2-pyridyl)benzol∗3HCl | 1-Naphtol | blau |
| **37** | 2,5-Diamino-1-(4-methyl-2-pyridyl)benzol∗3HCl | 5-Amino-2-methylphenol | rot |
| **38** | 2,5-Diamino-1-(4-methyl-2-pyridyl)benzol∗3HCl | Resorcin | dunkelblond |
| **39** | 2,5-Diamino-1-(5-trifluormethyl-2-pyridyl)-benzol∗3HCl | 1,3-Diamino-4-(2-hydroxyethoxy)-benzol∗sulfat | dunkelblau |
| **40** | 2,5-Diamino-1-(5-trifluormethyl-2-pyridyl)-benzol∗3HCI | 1-Naphtol | blau |
| **41** | 2,5-Diamino-1-(5-trifluormethyl-2-pyridyl)-benzol∗3HCl | 5-Amino-2-methyl-phenol | rot |
| **42** | 2,5-Diamino-1-(5-trifluormethyl-2-pyridyl)-benzol∗3HCl | Resorcin | dunkelblond |
| **43** | 2,5-Diamino-1-(5-nitro-2-pyridyl)-benzol∗3HCl | 1,3-Diamino-4-(2-hydroxyethoxy)-benzol∗sulfat | dunkelblau |
| **44** | 2,5-Diamino-1-(5-nitro-2-pyridyl)benzol∗3HCl | 1-Naphtol | blau |
| **45** | 2,5-Diamino-1-(5-nitro-2-pyridyl)benzol∗3HCl | 5-Amino-2-methyl-phenol | rot |
| **46** | 2,5-Diamino-1-(5-nitro-2-pyridyl)benzol∗3HCl | Resorcin | dunkelblond |
| **47** | 2,5-Diamino-1-(2-pyrimidyl)benzol∗3HCI | 1,3-Diamino-4-(2-hydroxyethoxy)-benzol∗sulfat | dunkelblau |
| **48** | 2,5-Diamino-1-(2-pyrimidyl)benzol∗3HCl | 1-Naphtol | blau |
| **49** | 2,5-Diamino-1-(2-pyrimidyl)benzol∗3HCl | 5-Amino-2-methyl-phenol | rot |
| **50** | 2,5-Diamino-1-(2-pyrimidyl)benzol∗3HCI | Resorcin | dunkelblond |

### Beispiel 51: Haarfärbemittel

Es wird eine Haarfärbelösung der folgenden Zusammensetzung hergestellt:

| | |
|---|---|
| 0,160 g | 2,5-Diamino-1-(2-pyridyl)benzol∗2HCl |
| 0,160 g | 1,4-Diamino-2-(2-hydroxyethyl)benzol∗sulfat |
| 0,137 g | 1,3-Dihydroxy-benzol |
| 0,100 g | 1,3-Dihydroxy-2-methylbenzol |
| 0,100 g | 2-Amino-5-methyl-phenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiel 52: Haarfärbemittel

Es wird eine Haarfärbelösung der folgenden Zusammensetzung hergestellt:

| | |
|---|---|
| 0,32 g | 2,5-Diamino-1-(2-pyridyl)benzol∗2HCl |
| 0,30 g | 5-Amino-2-methylphenol |
| 0,60 g | 4-Amino-3-methylphenol |
| 0,60 g | 4-Amino-phenol |
| 0,10 g | α-Naphtol |
| 0,20 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rote Färbung erhalten.

### Beispiel 53: Haarfärbemittel

Es wird eine Haarfärbelösung der folgenden Zusammensetzung hergestellt:

| | |
|---|---|
| 0,32 g | 2,5-Diamino-1-(2-pyridyl)benzol∗2HCl |
| 0,04 g | 5-Amino-2-methylphenol |
| 0,09 g | 2-Amino-4-(2'-hydroxyethyl)-amino-anisolsulfat |
| 0,03 g | 3-Aminophenol |
| 0,03 g | 1,3-Dihydroxy-benzol |
| 0,04 g | 1,3-Dihydroxy-2-methylbenzol |
| 0,10 g | 4-Amino-3-methylphenol |
| 0,20 g | 2-Amino-5-methylphenol |
| 0,10 g | 2-Amino-6-methylphenol-hydrochlorid |
| 0,01 g | 4-Chlor-N-(2-hydroxyethyl)-2-nitroanilin |
| 0,02 g | 2-Amino-4,6-dinitrophenol |
| 0,10 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 54: Haarfärbemittel

Es wird eine Haarfärbelösung der folgenden Zusammensetzung hergestellt:

| | |
|---|---|
| 0,32 g | 2,5-Diamino-1-(2-pyridyl)benzol∗2HCl |
| 0,04 g | 5-Amino-2-methylphenol |
| 0,05 g | 1,3-Diamino-4-(2-hydroxyethoxy)-benzol∗sulfat |
| 0,03 g | 3-Aminophenol |
| 0,03 g | 1,3-Dihydroxy-benzol |
| 0,04 g | 1,3-Dihydroxy-2-methylbenzol |
| 0,10 g | 4-Amino-3-methylphenol |
| 0,20 g | 2-Amino-5-methylphenol |
| 0,10 g | 2-Amino-6-methylphenol-hydrochlorid |
| 0,01 g | 4-Chlor-N-(2-hydroxyethyl)-2-nitroanilin |
| 0,02 g | 2-Amino-4,6-dinitrophenol |
| 0,10 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 55: Haarfärbemittel

Es wird eine Haarfärbelösung der folgenden Zusammensetzung hergestellt:

| | |
|---|---|
| 0,220 g | 2,5-Diamino-1-(2-pyridyl)benzol∗2HCl |
| 0,100 g | 1,4-Diamino-2-(2-hydroxyethyl)benzol∗sulfat |
| 0,020 g | 5-Amino-2-methylphenol |
| 0,010 g | 1,3-Diamino-4-(2-hydroxyethoxy)-benzol∗sulfat |
| 0,004 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-3-methylphenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 56: Haarfärbemittel

Es wird eine Haarfärbelösung der folgenden Zusammensetzung hergestellt:

| | |
|---|---|
| 0,220 g | 2,5-Diamino-1-(2-pyridyl)benzol∗2HCl |
| 0,100 g | 4-Di-(2-hydroxyethyl)amino-anilin-sulfat |
| 0,020 g | 5-Amino-2-methylphenol |
| 0,010 g | 1,3-Diamino-4-(2-hydroxyethoxy)-benzol∗sulfat |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4,5-Diamino-1-(2-hydroxyethyl)-1 H-pyrazol-sulfat |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 57: Haarfärbemittel

Es wird eine Haarfärbelösung der folgenden Zusammensetzung hergestellt:

| | |
|---|---|
| 0,320 g | 2,5-Diamino-1-(2-pyridyl)benzol∗2HCl |
| 0,020 g | 5-Amino-2-methylphenol |
| 0,010 g | 1,3-Diamino-4-(2-hydroxyethoxy)-benzol∗sulfat |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-2-(aminomethyl)phenol-dihydrochlorid |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiele 58:

Es wird eine Haarfärbelösung der folgenden Zusammensetzung hergestellt:

| | |
|---|---|
| 0,600 g | 2,5-Diamino-1-(2-pyridyl)benzol∗2HCl |
| 0,137 g | 1,3-Dihydroxy-benzol |
| 0,150 g | 1-Chlor-2,4-dihydroxybenzol |
| 0,100 g | 3-Aminophenol |
| 10,000 g | Kaliumoleat (8prozentige wäßrige Lösung) |
| 10,000 g | Ammoniak (22prozentige wäßrige Lösung) |
| 10,000 g | Ethanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine mittelblonde Färbung erhalten.

### Beispiele 59:

Es wird eine Haarfärbelösung der folgenden Zusammensetzung hergestellt:

| | |
|---|---|
| 0,600 g | 2,5-Diamino-1-(3-methyl-2-pyridyl)benzol∗2HCl |
| 0,137 g | 1,3-Dihydroxy-benzol |
| 0,150 g | 1-Chlor-2,4-dihydroxybenzol |
| 0,100 g | 5-Amino-2-methylphenol |
| 10,000 g | Kaliumoleat (8prozentige wäßrige Lösung) |
| 10,000 g | Ammoniak (22prozentige wäßrige Lösung) |
| 10,000 g | Ethanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine mittleblonde Färbung erhalten.

### Beispiele 60:

Es wird eine Haarfärbelösung der folgenden Zusammensetzung hergestellt:

| | |
|---|---|
| 0,40 g | 2,5-Diamino-1-(2-pyridyl)benzol∗2HCl |
| 0,20g | 1-Chlor-2,4-dihydroxybenzol |
| 0,05 g | 3-Aminophenol |
| 0,09 g | 1,3-Diamino4-(2-hydroxyethoxy)benzol sulfat |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 61:

Es wird eine Haarfärbelösung der folgenden Zusammensetzung hergestellt:

| | |
|---|---|
| 0,40 g | 2,5-Diamino-1-(3-methyl2-pyridyl)benzol∗2HCl |
| 0,20 g | 1-Chlor-2,4-dihydroxybenzol |
| 0,05 g | 2-Amino-5-methylphenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 62:

Es wird eine Haarfärbelösung der folgenden Zusammensetzung hergestellt:

| | |
|---|---|
| 0,52 g | 2,5-Diamino-1-(2-pyridyl)benzol∗2HCl |
| 0,70 g | 5-Hydroxy-1,3-benzodioxol |
| 0,05 g | 3-Aminophenol |
| 15,00 g | Cetylalkohol |
| 3,00 g | Ammoniak (22prozentige wäßrige Lösung) |
| 3,50 g | Natrium-laurylalkohol-diglykolethersulfat (28prozentige wäßrige Lösung) |
| 0,30 g | Natriumsulfit, wasserfrei |
| ad 100,00 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten wird das Haar mit Wasser gespült, mit einem handelsüblichen Shampoo gewaschen und getrocknet. Das Haar hat eine blonde Färbung erhalten.

### Beispiele 63:

Es wird eine Haarfärbelösung der folgenden Zusammensetzung hergestellt:

| | |
|---|---|
| 0,52 g | 2,5-Diamino-1-(3-methyl-2-pyridyl)benzol∗2HCl |
| 0,70 g | 5-Hydroxy-1,3-benzodioxol |
| 0,05 g | 3-Amino-2-chlor-6-methylphenol |
| 15,00 g | Cetylalkohol |
| 3,00 g | Ammoniak (22prozentige wäßrige Lösung) |
| 3,50 g | Natrium-laurylalkohol-diglykolethersulfat (28prozentige wäßrige Lösung) |
| 0,30 g | Natriumsulfit, wasserfrei |
| ad 100,00 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei wird das Haar mit Wasser gespült, mit einem handelsüblichen Shampoo gewaschen und getrocknet. Das Haar hat eine mittelblonde Färbung erhalten.

### Beispiele 64:

Es wird eine Haarfärbelösung der folgenden Zusammensetzung hergestellt:

| | |
|---|---|
| 0,40 g | 2,5-Diamino-1-(2-pyridyl)benzol∗2HCl |
| 0,20 g | 5-((2-Hydroxyethyl)amino)-1,3-benzodioxol-hydrochlorid |
| 0,05 g | 3-Aminophenol |
| 0,10 g | 3-Amino-2-chlor-6-methylphenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 65:

Es wird eine Haarfärbelösung der folgenden Zusammensetzung hergestellt:

| | |
|---|---|
| 0,40 g | 2,5-Diamino-1-(3-methyl-2-pyridyl)benzol∗2HCl |
| 0,20 g | 5-((2-Hydroxyethyl)amino)-1,3-benzodioxol-hydrochlorid |
| 0,05 g | 5-Amino-2-methylphenol |
| 0,10 g | 3-Amino-2-chlor-6-methylphenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 66:

Es wird eine Haarfärbelösung der folgenden Zusammensetzung hergestellt:

| | |
|---|---|
| 1,02 g | 2,5-Diamino-1-(2-pyridyl)benzol∗2HCl |
| 0,50 g | 1,3-Dihydroxybenzol |
| 0,09 g | 3-Aminophenol |
| 0,01 g | 2,4-Diamino-1-1fluor-5-methylbenzol-sulfat |
| 15,00 g | Cetylalkohol |
| 3,00 g | Ammoniak (22prozentige wäßrige Lösung) |
| 3,50 g | Natrium-laurylalkohol-diglykolethersulfat (28prozentige wäßrige Lösung) |
| 0,30 g | Natriumsulfit, wasserfrei |
| ad 100,00 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiele 67:

Es wird eine Haarfärbelösung der folgenden Zusammensetzung hergestellt:

| | |
|---|---|
| 1,02 g | 2,5-Diamino-1-(3-methyl-2-pyridyl)benzol*2HCl |
| 0,50 g | 1,3-Dihydroxy-2-methylbenzol |
| 0,09 g | 5-Amino-2-methylphenol |
| 0,01 g | 2,4-Diamino-1-1fluor-5-methylbenzol-sulfat |
| 15,00 g | Cetylalkohol |
| 3,00 g | Ammoniak (22prozentige wäßrige Lösung) |
| 3,00 g | Natrium-laurylalkohol-diglykolethersulfat (28prozentige wäßrige Lösung) |
| 0,30 g | Natriumsulfit, wasserfrei |
| ad 100,00 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braunee Färbung erhalten.

### Beispiel 68: Haarfärbemittel

Es wird eine Haarfärbelösung der folgenden Zusammensetzung hergestellt:

| | |
|---|---|
| 0,62 g | 2,5-Diamino-1-(2-pyridyl)benzol∗2HCl |
| 0,15 g | 3-Amino-6-methoxy-2-(methylamino)pyridin-dihydrochlorid |
| 0,05 g | 1,3-Diamino-4-(2-hydroxyethoxy)benzol-hydrochlorid |
| 0,30 g | 1,3-Dihydroxy-benzol |
| 0,10 g | 4-Amino-5-methylphenol |
| 10,0 g | Kaliumoleat (8prozentige wäßrige Lösung) |
| 10,0 g | Ammoniak (22prozentige wäßrige Lösung) |
| 10,0 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 69: Haarfärbemittel

Es wird eine Haarfärbelösung der folgenden Zusammensetzung hergestellt:

| | |
|---|---|
| 0,62 g | 2,5-Diamino-1-(3-methyl-2-pyridyl)benzol∗2HCl |
| 0,05 g | 3-Amino-6-methoxy-2-(methylamino)pyridin-dihydrochlorid |
| 0,30 g | 1-Chlor-2,4-dihydroxy-benzol |
| 0,10 g | 3-Aminophenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 70: Haarfärbemittel

Es wird eine Haarfärbelösung der folgenden Zusammensetzung hergestellt:

| | |
|---|---|
| 0,50 g | 2,5-Diamino-1-(3-methyl-2-pyridyl)benzol∗2HCl |
| 0,20 g | 5-Methyl-2-(1-methylethyl)phenol |
| 0,30 g | 1,3-Dihydroxy-benzol |
| 0,05 g | 4-Amino-5-methylphenol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiel 71: Haarfärbemittel

Es wird eine Haarfärbelösung der folgenden Zusammensetzung hergestellt:

| | |
|---|---|
| 0,62 g | 2,5-Diamino-1-(2-pyridyl)benzol∗2HCl |
| 0,06 g | 5-Methyl-2-(1-methylethyl)phenol |
| 0,30 g | 1,3-Dihydroxy-benzol |
| 0,150 g | 1,3-Dihydroxy-2-methylbenzol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 72:

Es wird eine Haarfärbelösung der folgenden Zusammensetzung hergestellt:

| | |
|---|---|
| 3,02 g | 2,5-Diamino-1-(3-methyl-2-pyridyl)benzol∗2HCl |
| 1,50 g | 1,3-Dihydroxybenzol |
| 1,50 g | 1,3-Diamino-4-(2-hydroxyethoxy)benzol-sulfat |
| 0,30 g | 3-Aminophenol |
| 15,00 g | Cetylalkohol |
| 3,00 g | Ammoniak (22prozentige wäßrige Lösung) |
| 3,50 g | Natrium-laurylalkohol-diglykolethersulfat (28prozentige wäßrige Lösung) |
| 0,30 g | Natriumsulfit, wasserfrei |
| ad 100,0 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten wird das Haar mit Wasser gespült, mit einem handelsüblichen Shampoo gewaschen und getrocknet. Das Haar hat eine svwarze Färbung erhalten.

### Beispiele 73:

Es wird eine Haarfärbelösung der folgenden Zusammensetzung hergestellt:

| | |
|---|---|
| 3,02 g | 2,5-Diamino-1-(2-pyridyl)benzol∗2HCl |
| 1,50 g | 1,3-Dihydroxybenzol |
| 1,50 g | 5-((2-hydroxyethyl)amino)-2-methoxy-anilin-sulfat |
| 0,30 g | 3-Aminophenol |
| 15,00 g | Cetylalkohol |
| 3,00 g | Ammoniak (22prozentige wäßrige Lösung) |
| 3,50 g | Natrium-laurylalkohol-diglykolethersulfat (28prozentige wäßrige Lösung) |
| 0,30 g | Natriumsulfit, wasserfrei |
| ad 100,00 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten wird das Haar mit Wasser gespült, mit einem handelsüblichen Shampoo gewaschen und getrocknet. Das Haar hat eine schwarze Färbung erhalten.

### Beispiel 74: Haarfärbemittel

Es wird eine Haarfärbelösung der folgenden Zusammensetzung hergestellt:

| | |
|---|---|
| 0,30 g | 2,5-Diamino-1-(3-methyl-2-pyridyl)benzol∗2HCl |
| 0,18 g | 1-Chlor-2,4-dihydroxybenzol |
| 0,30 g | 4,5-Diamino-1-(2'-hydroxyethyl)-1H-pyrazol-sulfat |
| 0,30 g | 1-Naphtol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 75: Haarfärbemittel

Es wird eine Haarfärbelösung der folgenden Zusammensetzung hergestellt:

| | |
|---|---|
| 0,30 g | 2,5-Diamino-1-(2-pyridyl)benzol∗2HCl |
| 0,18 g | 1,3-Dihydroxy-2-methylbenzol |
| 0,30 g | 4-Amino-2-(aminomethyl)phenol-hydrochloride |
| 0,30 g | 1-Naphtol |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 76: Haarfärbemittel

Es wird eine Haarfärbelösung der folgenden Zusammensetzung hergestellt:

| | |
|---|---|
| 0,30 g | 2,5-Diamino-1-(3-methyl-2-pyridyl)benzol∗2HCl |
| 0,18 g | 1,3-Dihydroxybenzol |
| 0,3 g | 4,5-Diamino-1-(2'-hydroxyethyl)-1H-pyrazol-sulfat |
| 0,3g | Essigsäure-(-methylnaphthalin-1-yl)ester |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 77: Haarfärbemittel

Es wird eine Haarfärbelösung der folgenden Zusammensetzung hergestellt:

| | |
|---|---|
| 0,30 g | 2,5-Diamino-1-(2-pyridyl)benzol∗2HCl |
| 0,18 g | 1,3-Dihydroxybenzol |
| 0,30 g | 4-Amino-3methylphenol |
| 0,30 g | Essigsäure-(-methylnaphthalin-1-yl)ester |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rotbraune Färbung erhalten.

### Beispiel 78: Haarfärbemittel

Es wird eine Haarfärbelösung der folgenden Zusammensetzung hergestellt:

| | |
|---|---|
| 1,30 g | 2,5-Diamino-1-(3-methyl-2-pyridyl)benzol∗2HCl |
| 0,08 g | 5-Amino-2-methylphenol |
| 0,05 g | 1,3-Diamino-4-(2-hydroxyethoxy)-benzol∗sulfat |
| 0,01 g | 3-Aminophenol |
| 0,50 g | 1,3-Dihydroxy-benzol |
| 0,10 g | 4-Amino-5-methylphenol |
| 0,02 g | 2-Amino-5-methylphenol |
| 0,02 g | 2-amino-6-chlor-4-nitro-phenol-hydrochlorid |
| 10,00 g | Kaliumoleat (8prozentige wäßrige Lösung) |
| 10,00 g | Ammoniak (22prozentige wäßrige Lösung) |
| 10,00 g | Isopropanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiele 79:

Es wird eine Haarfärbelösung der folgenden Zusammensetzung hergestellt:

| | |
|---|---|
| 0,60 g | 2,5-Diamino-1-(2-pyridyl)benzol∗2HCl |
| 0,30 g | 1,3-Dihydroxy-benzol |
| 0,01 g | 2-Amino-5-methylphenol |
| 0,10 g | 3-Amino-2-chlor-6-methylphenol |
| 0,05 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiele 80:

Es wird eine Haarfärbelösung der folgenden Zusammensetzung hergestellt:

| | |
|---|---|
| 0,90 g | 2,5-Diamino-1-(3-methyl-2-pyridyl)benzol∗2HCl |
| 0,40 g | 1,3-Dihydroxy-benzol |
| 0,01 g | 2-Amino-5-methylphenol |
| 0,10 g | 3-Aminophenol |
| 0,02 g | 2-Amino-6-chlor-4-nitro-phenol-hydrochlorid |
| 0,01 g | 2-Amino-4,6-dinitrophenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiele 81:

Es wird eine Haarfärbelösung der folgenden Zusammensetzung hergestellt:

| | |
|---|---|
| 0,90 g | 2,5-Diamino-1-(2-pyridyl)benzol∗2HCl |
| 0,40 g | 1,3-Dihydroxy-benzol |
| 0,40 g | 1,3-Dihydroxy-2-methylphenol |
| 0,10 g | 5-Amino-2-methylphenol |
| 0,05 g | 3-Amino-2-Chlor-6-methylphenol |
| 0,40 g | 1,4-Diamino-2-(hydroxyethyl)benzol sulfat |
| 0,05 g | 4-Amino-3-methylphenol-hydrochlorid |
| 0,01 g | 2-Amino-4,6-dinitrophenol |
| 0,07 g | 2-Chlor-6-(ethylamino)-4-nitrophenol |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiele 82:

Es wird eine Haarfärbelösung der folgenden Zusammensetzung hergestellt:

| | |
|---|---|
| 0,90 g | 2,5-Diamino-1-(2-pyridyl)benzol∗2HCl |
| 0,50 g | 2,5-Diamino-1-methylbenzol sulfat |
| 0,40 g | 1,3-Dihydroxy-benzol |
| 0,40 g | 4-Amino-3-methylphenol |
| 0,10 g | 5-Amino-2-methylphenol |
| 0,10 g | 5-((2-Hydroxyethyl)amino-2-methoxyanilin sulfat |
| 0,10 g | 3-Aminophenol |
| 0,05 g | 1,3-Diamino-4-(2-hydroxyethoxybenzol-sulfat |
| 0,07 g | 2-Amino-6-chlor-4-nitro-phenol-hydrochlorid |
| 10,00 g | Kaliumoleat (8prozentige wäßrige Lösung) |
| 10,00 g | Ammoniak (22prozentige wäßrige Lösung) |
| 10,00 g | Ethanol |
| 0,30 g | Ascorbinsäure |
| ad 100,00 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

Alle Gewichtsangaben stellen, soweit nicht anders angegeben, Gewichtsprozente dar.

## Patentansprüche

1. p-Diaminobenzol-Derivat, das ausgewählt ist aus 2,5-Diamino-1-(2-pyridyl)benzol; 2,5-Diamino-1-(3-pyridyl)benzol; 2,5-Diamino-1-(4-pyridyl)-benzol; 2,5-Diamino-4-methoxy-1-(2-pyridyl)benzol; 2,5-Diamino-4-methoxy-1-(3-pyridyl)benzol; 2,5-Diamino-4-methoxy-1-(4-pyridyl)benzol; 2,5-Diamino-4-methyl-1-(2-pyridyl)benzol; 2,5-Diamino-4-methyl-1-(3-pyridyl)benzol; 2,5-Diamino-4-methyl-1-(4-pyridyl)benzol; 2,5-Diamino-1-(3-amino-2-pyridyl)benzol; 2,5-Dlamino-1-(3-chlor-2-pyridyl)benzol; 2,5-Diamino-1-(3-fluor-2-pyridyl)benzol; 2,5-Diamino-1-(3-hydroxy-2-pyridyl)benzol; 2,5-Diamino-1-(3-methyl-2-pyridyl)benzol; 2,5-Diamino-1-(3-trifluormethyl-2-pyridyl)benzol; 2,5-Diamino-1-(4-amino-2-pyridyl)-benzol; 2,5-Diamino-1-(4-chlor-2-pyridyl)benzol; 2,5-Diamino-1-(4-fluor-2-pyridyl)benzol; 2,5-Diamino-1-(4-hydroxy-2-pyridyl)benzol; 2,5-Diamino-1-(4-methyl-2-pyridyl)benzol; 2,5-Diamino-1-(4-trifluormethyl-2-pyridyl)-benzol; 2,5-Diamino-1-(5-amino-2-pyridyl)benzol; 2,5-Diamino-1-(5-chlor-2-pyridyl)benzol; 2,5-Diamino-1-(5-fluor-2-pyridyl)benzol; 2,5-Diamino-1-(5-hydroxy-2-pyridyl)benzol; 2,5-Diamino-1-(5-methyl-2-pyridyl)benzol; 2,5-Diamino-1-(5-trifluormethyl-2-pyridyl)benzol; 2,5-Diamino-1-(6-amino-2-pyridyl)benzol; 2,5-Diamino-1-(6-chlor-2-pyridyl)benzol; 2,5-Diamino-1-(6-fluor-2-pyridyl)benzol; 2,5-Diamino-1-(6-hydroxy-2-pyridyl)benzol; ,5-Diamino-1-(6-methyl-2-pyridyl)benzol; 2,5-Diamino-1-(6-trifluormethyl-2-pyridyl)benzol; 2,5-Diamino-1-(2-amino-3-pyridyl)benzol; 2,5-Diamino-1-(2-chlor-3-pyridyl)benzol; 2,5-Diamino-1-(2-fluor-3-pyridyl)benzol; 2,5-Diamino-1-(2-hydroxy-3-pyridyl)benzol; 2,5-Diamino-1-(2-methyl-3-pyridyl)benzol; 2,5-Diamlno-1-(2-trifluormethyl-3-pyridyl)benzol; 2,5-Diamino-1-(4-amino-3-pyridyl)benzol; 2,5-Diamino-1-(4-chlor-3-pyridyl)benzol; 2,5-Diamino-1-(4-fluor-3-pyridyl)benzol; 2,5-Diamino-1-(4-hydroxy-3-pyridyl)benzol; 2,5-Diamino-1-(4-methyl-3-pyridyl)benzol; 2,5-Diamino-1-(4-trifluormethyl-3-pyridyl)benzol; 2,5-Diamino-1-(5-amino-3-pyridyl)benzol; 2,5-Diamino-1-(5-chlor-3-pyridyl)benzol; 2,5-Diamino-1-(5-fluor-3-pyridyl)benzol; 2,5-Diamino-1-(5-hydroxy-3-pyridyl)benzol; 2,5-Diamino-1-(5-methyl-3-pyridyl)benzol; 2,5-Diamino-1-(5-trifluormethyl-3-pyridyl)benzol; 2,5-Diamino-1-(6-amino-3-pyridyl)benzol; 2,5-Diamino-1-(6-chlor-3-pyridyl)benzol; 2,5-Diamino-1-(6-fluor-3-pyridyl)benzol; 2,5-Diamino-1-(6-hydroxy-3-pyridyl)benzol; 2,5-Diamino-1-(6-methyl-3-pyridyl)benzol; 2,5-Diamino-1-(6-trifluormethyl-3-pyridyl)benzol; 2,5-Diamino-1-(2-amino-4-pyridyl)benzol; 2,5-Diamino-1-(2-chlor-4-pyridyl)benzol; 2,5-Diamino-1-(2-fluor-4-pyridyl)benzol; 2,5-Diamino-1-(2-hydroxy-4-pyridyl)benzol; 2,5-Diamino-1-(2-methyl-4-pyridyl)benzol; 2,5-Diamino-1-(2-trifluormethyl-4-pyridyl)benzol; 2,5-Diamino-1-(3-amino-4-pyridyl)benzol; 2,5-Diamino-1-(3-chlor-4-pyridyl)benzol; 2,5-Diamino-1-(3-fluor-4-pyridyl)benzol; 2,5-Diamino-1-(3-hydroxy-4-pyridyl)benzol; 2,5-Diamino-1-(3-methyl-4-pyridyl)benzol; 2,5-Diamino-1-(3-trifluormethyl-4-pyridyl)benzol; 2,5-Diamino-1-(5-amino-4-pyridyl)benzol; 2,5-Diamino-1-(5-chlor-4-pyridyl)benzol; 2,5-Diamino-1-(5-fluor-4-pyridyl)benzol; 2,5-Diamino-1-(5-hydroxy-4-pyridyl)benzol; 2,5-Diamino-1-(5-methyl-4-pyridyl)benzol; 2,5-Diamino-1-(5-trilluormethyl-4-pyridyl)benzol; 2,5-Diamino-1-(6-amino-4-pyridyl)benzol; 2,5-Diamino-1-(6-chlor-4-pyridyl)benzol; 2,5-Diamino-1-(6-fluor-4-pyridyl)benzol; 2,5-Diamino-1-(6-hydroxy-4-pyridyl)benzol; 2,5-Diamino-1-(6-methyl-4-pyridyl)benzol; 2,5-Diamino-1-(6-trifluormethyl-4-pyridyl)benzol; 2,5-Diamino-1-(2-pyrimidyl)benzol; 2,5-Diamino-1-(4-pyrimidyl)benzol; 2,5-Diamino-1-(5-pyrimidyl)benzol; 2,5-Diamino-1-(6-pyrimidyl)benzol; 2-Hydroxyethylamino-5-amino-1-(2-pyridyl)benzol; 2-Hydroxyethylamino-5-amino-1-(2-pyridyl)benzol; 2-Hydroxyethylamino-5-amino-1-(3-pyridyl)benzol; 2-Hydroxyethylamino-5-amino-1-(4-pyridyl)benzol; 2-Bis(hydroxyethyl)-amino-5-amino-1-(2-pyridyl)benzol; 2-Bis(hydroxyethyl)amino-5-amino-1-(3-pyridyl)benzol; 2-Bis(hydroxyethyl)amino-5-amino-1-(4-pyridyl)benzol; 2,5-Diamino-1-(3-cyan-2-pyridyl)benzol; 2,5-Diamino-1-(3-nitro-2-pyridyl)benzol; 2,5-Diamino-1-(4-cyan-2-pyridyl)benzol; 2,5-Diamino-1-(4-nitro-2-pyridyl)benzol; 2,5-Diamino-1-(5-cyan-2-pyridyl)benzol; 2,5-Diamino-1-(5-nitro-2-pyridyl)benzol; 2,5-Diamino-1-(6-cyan-2-pyridyl)benzol; 2,5-Diamino-1-(6-nitro-2-pyridyl)benzol; 2,5-Diamino-1-(2-cyan-4-pyridyl)benzol; 2,5-Diamino-1-(2-nitro-4-pyridyl)benzol; 2,5-Diamino-1-(3-cyan-4-pyridyl)benzol; 2,5-Diamino-1-(3-nitro-4-pyridyl)benzol; 2,5-Diamino-1-(5-cyan-4-pyridyl)benzol; 2,5-Diamino-1-(5-nitro-4-pyridyl)benzol; 2,5-Diamino-1-(6-cyan-4-pyridyl)benzol; 2,5-Diamino-1-(6-nitro-4-pyridyl)benzol; 2,5-Diamino-1-(2-cyan-3-pyridyl)benzol; 2,5-Diamino-1-(2-nitro-3-pyridyl)benzol; 2,5-Diamino-1-(4-cyan-3-pyridyl)benzol; 2,5-Diamino-1-(4-nitro-3-pyridyl)benzol; 2,5-Diamino-1-(5-cyan-3-pyridyl)benzol; 2,5-Diamino-1-(5-nitro-3-pyridyl)benzol; 2,5-Diamino-1-(6-cyan-3-pyridyl)benzol und 2,5-Diamino-1-(6-nitro-3-pyridyl)benzol oder deren physiologisch verträglichen Salzen.

2. p-Diaminobenzol-Derivat nach Anspruch 1, **dadurch gekennzeichnet, daß** es ausgewählt ist aus 2,5-Diamino-1-(2-pyridyl)-benzol; 2,5-Diamino-1-(3-pyridyl)benzol; 2,5-Diamino-1-(4-pyridyl)benzol; 2,5-Diamino-1-(2-pyrimidyl)benzol; 2,5-Diamino-1-(6-methyl-2-pyridyl)-benzol; 2,5-Diamino-1-(4-methyl-2-pyridyl)benzol; 2,5-Diamino-1-(5-methyl-2-pyridyl)benzol und 2,5-Diamino-1-(3-methyl-2-pyridyl)benzol oder deren physiologisch verträglichen Salzen.

3. Mittel zum oxidativen Färben von Keratinfasern, auf der Basis einer Entwicklersubstanz-Kupplersubstanz-Kombination, **dadurch gekennzeichnet, daß** es als Entwicklersubstanz mindestens ein Diaminobenzol-Derivat nach Anspruch 1oder 2 enthält.

4. Mittel nach Anspruch 3, **dadurch gekennzeichnet, daß** das Diaminobenzol-Derivat in einer Menge von 0,005 bis 20 Gewichtsprozent enthalten ist.

5. Mittel nach Anspruch 3 oder 4, **dadurch gekennzeichnet, daß** die Entwicklersubstanzen und Kupplersubstanzen, bezogen auf die Gesamtmenge des Oxidationsfärbemittel jeweils in einer Gesamtmenge von 0,005 bis 20 Gewichtsprozent enthalten sind,

6. Mittel nach einem der Ansprüche 3 bis 5, **dadurch gekennzeichnet, daß** es zusätzlich mindestens einen direktziehenden Farbstoff enthält.

7. Mittel nach einem der Ansprüche 3 bis 6, **dadurch gekennzeichnet, daß** es einen pH-Wert von 6,8 bis 11,5 aufweist.

8. Mittel einem der Ansprüche 3 bis 7, **dadurch gekennzeichnet, daß** es außer dem 1,4-Diaminobenzol-Derivat nach Anspruch 1 oder 2 zusätzlich mindestens eine weitere Entwicklersubstanz aus der Gruppe bestehend aus 1,4-Diaminobenzol, 2,5-Diaminotoluol, 2,5-Diaminophenylethanol, 4-Aminophenol und seinen Derivaten, 4,5-Diaminopyrazolderivaten und Tetraaminopyrimidinen enthält.

9. Mittel nach einem der Ansprüche 3 bis 8, **dadurch gekennzeichnet, daß** es in Form einer wäßrigen oder wäßrig-alkoholischen Lösung, einer Creme, eines Gels oder einer Emulsion vorliegt.

10. Mittel nach einem der Ansprüche 3 bis 9, **dadurch gekennzeichnet, daß** es ein Haarfärbemittel ist.

## Claims

1. p-Diaminobenzene derivative chosen from 2,5-diamino-1-(2-pyridyl)benzene; 2,5-diamino-1-(3-pyridyl)benzene; 2,5-diamino-1-(4-pyridyl)benzene; 2,5-diamino-4-methoxy-1-(2-pyridyl)benzene; 2,5-diamino-4-methoxy-1-(3-pyridyl)benzene; 2,5-diamino-4-methoxy-1-(4-pyridyl)benzene; 2,5-diamino-4-methyl-1-(2-pyridyl)-benzene; 2,5-diamino-4-methyl-1-(3-pyridyl)benzene; 2,5-diamino-4-methyl-1-(4-pyridyl)benzene; 2,5-diamino-1-(3-amino-2-pyridyl)benzene; 2,5-diamino-1-(3-chloro-2-pyridyl)benzene; 2,5-diamino-1-(3-fluoro-2-pyridyl)-benzene; 2,5-diamino-1-(3-hydroxy-2-pyridyl)benzene; 2,5-diamino-1-(3-methyl-2-pyridyl)benzene; 2,5-diamino-1-(3-trifluoromethyl-2-pyridyl)benzene; 2,5-diamino-1-(4-amino-2-pyridyl)benzene; 2,5-diamino-1-(4-chloro-2-pyridyl(benzene; 2,5-diamino-1-(4-fluoro-2-pyridyl)-benzene; 2,5-diamino-1-(4-hydroxy-2-pyridyl)benzene; 2,5-diamino-1-(4-methyl-2-pyridyl)benzene; 2,5-diamino-1-(4-trifluoromethyl-2-pyridyl)benzene; 2,5-diamino-1-(5-amino-2-pyridyl)benzene; 2,5-diamino-1-(5-chloro-2-pyridyl)benzene; 2,5-diamino-1-(5-fluoro-2-pyridyl)-benzene; 2,5-diamino-1-(5-hydroxy-2-pyridyl)benzene; 2,5-diamino-1-(5-methyl-2-pyridyl)benzene; 2,5-diamino-1-(5-trifluoromethyl-2-pyridyl)benzene; 2,5-diamino-1-(6-amino-2-pyridyl)benzene; 2,5-diamino-1-(6-chloro-2-pyridyl)benzene; 2,5-diamino-1-(6-fluoro-2-pyridyl)-benzene; 2,5-diamino-1-(6-hydroxy-2-pyridyl)benzene; 2,5-diamino-1-(6-methyl-2-pyridyl)benzene; 2,5-diamino-1-(6-trifluoromethyl-2-pyridyl)benzene; 2,5-diamino-1-(2-amino-3-pyridyl)benzene; 2,5-diamino-1-(2-chloro-3-pyridyl)benzene; 2,5-diamino-1-(2-fluoro-3-pyridyl)-benzene; 2,5-diamino-1-(2-hydroxy-3-pyridyl)benzene; 2,5-diamino-1-(2-methyl-3-pyridyl)benzene; 2,5-diamino-1-(2-trifluoromethyl-3-pyridyl)benzene; 2,5-diamino-1-(4-amino-3-pyridyl)benzene; 2,5-diamino-1-(4-chloro-3-pyridyl)benzene; 2,5-diamino-1-(4-fluoro-3-pyridyl)-benzene; 2,5-diamino-1-(4-hydroxy-3-pyridyl)benzene; 2,5-diamino-1-(4-methyl-3-pyridyl)benzene; 2,5-diamino-1-(4-trifluoromethyl-3-pyridyl)benzene; 2,5-diamino-1-(5-amino-3-pyridyl)benzene; 2,5-diamino-1-(5-chloro-3-pyridyl)benzene; 2,5-diamino-1-(5-fluoro-3-pyridyl)-benzene; 2,5-diamino-1-(5-hydroxy-3-pyridyl)benzene; 2,5-diamino-1-(5-methyl-3-pyridyl)benzene; 2,5-diamino-1-(5-trifluoromethyl-3-pyridyl)benzene; 2,5-diamino-1-(6-amino-3-pyridyl)benzene; 2,5-diamino-1-(6-chloro-3-pyridyl)benzene; 2,5-diamino-1-(6-fluoro-3-pyridyl)-benzene; 2,5-diamino-1-(6-hydroxy-3-pyridyl)benzene; 2,5-diamino-1-(6-methyl-3-pyridyl)benzene; 2,5-diamino-1-(6-trifluoromethyl-3-pyridyl)benzene; 2,5-diamino-1-(2-amino-4-pyridyl)benzene; 2,5-diamino-1-(2-chloro-4-pyridyl)benzene; 2,5-diamino-1-(2-fluoro-4-pyridyl)-benzene; 2,5-diamino-1-(2-hydroxy-4-pyridyl)benzene; 2,5-diamino-1-(2-methyl-4-pyridyl)benzene; 2,5-diamino-1-(2-trifluoromethyl-4-pyridyl)benzene; 2,5-diamino-1-(3-amino-4-pyridyl)benzene; 2,5-diamino-1-(3-chloro-4-pyridyl)benzene; 2,5-diamino-1-(3-fluoro-4-pyridyl)-benzene; 2,5-diamino-1-(3-hydroxy-4-pyridyl)benzene; 2,5-diamino-1-(3-methyl-4-pyridyl)benzene; 2,5-diamino-1-(3-trifluoromethyl-4-pyridyl)benzene; 2,5-diamino-1-(5-amino-4-pyridyl)benzene; 2,5-diamino-1-(5-chloro-4-pyridyl)benzene; 2,5-diamino-1-(5-fluoro-4-pyridyl)-benzene; 2,5-diamino-1-(5-hydroxy-4-pyridyl)benzene; 2,5-diamino-1-(5-methyl-4-pyridyl)benzene; 2,5-diamino-1-(5-trifluoromethyl-4-pyridyl)benzene; 2,5-diamino-1-(6-amino-4-pyridyl)benzene; 2,5-diamino-1-(6-chloro-4-pyridyl)benzene; 2,5-diamino-1-(6-fluoro-4-pyridyl)-benzene; 2,5-diamino-1-(6-hydroxy-4-pyridyl)benzene; 2,5-diamino-1-(6-methyl-4-pyridyl)benzene; 2,5-diamino-1-(6-trifluoromethyl-4-pyridyl)benzene; 2,5-diamino-1-(2-pyrimidyl)benzene; 2,5-diamino-1-(4-pyrimidyl)-benzene; 2,5-diamino-1-(5-pyrimidyl)benzene; 2,5-diamino-1-(6-pyrimidyl)benzene; 2-hydroxyethylamino-5-amino-1-(2-pyridyl)benzene; 2-hydroxyethylamino-5-amino-1-(2-pyridyl)benzene; 2-hydroxyethylamino-5-amino-1-(3-pyridyl)benzene; 2-hydroxyethylamino-5-amino-1-(4-pyridyl)benzene; 2-bis(hydroxyethyl)amino-5-amino-1-(2-pyridyl)benzene; 2-bis(hydroxyethyl)amino-5-amino-1-(3-pyridyl)benzene; 2-bis(hydroxyethyl)amino-5-amino-1-(4-pyridyl)benzene; 2,5-diamino-1-(3-cyano-2-pyridyl)benzene; 2,5-diamino-1-(3-nitro-2-pyridyl)-benzene; 2,5-diamino-1-(4-cyano-2-pyridyl)benzene; 2,5-diamino-1-(4-nitro-2-pyridyl)benzene; 2,5-diamino-1-(5-cyano-2-pyridyl)benzene; 2,5-diamino-1-(5-nitro-2-pyridyl)benzene; 2,5-diamino-1-(6-cyano-2-pyridyl)benzene; 2,5-diamino-1-(6-nitro-2-pyridyl)-benzene; 2,5-diamino-1-(2-cyano-4-pyridyl)benzene; 2,5-diamino-1-(2-nitro-4-pyridyl)benzene; 2,5-diamino-1-(3-cyano-4-pyridyl)benzene; 2,5-diamino-1-(3-nitro-4-pyridyl)benzene; 2,5-diamino-1-(5-cyano-4-pyridyl)-benzene; 2,5-diamino-1-(5-nitro-4-pyridyl)benzene; 2,5-diamino-1-(6-cyano-4-pyridyl)benzene; 2,5-diamino-1-(6-nitro-4-pyridyl)benzene; 2,5-diamino-1-(2-cyano-3-pyridyl)benzene; 2,5-diamino-1-(2-nitro-3-pyridyl)-benzene; 2,5-diamino-1-(4-cyano-3-pyridyl)benzene; 2,5-diamino-1-(4-nitro-3-pyridyl)benzene; 2,5-diamino-1-(5-cyano-3-pyridyl)benzene; 2,5-diamino-1-(5-nitro-3-pyridyl)benzene; 2,5-diamino-1-(6-cyano-3-pyridyl)-benzene and 2,5-diamino-1-(6-nitro-3-pyridyl)benzene or physiologically compatible salts thereof.

2. p-Diaminobenzene derivative according to Claim 1, **characterized in that** it is chosen from 2,5-diamino-1-(2-pyridyl)benzene; 2,5-diamino-1-(3-pyridyl)benzene; 2,5-diamino-1-(4-pyridyl)benzene; 2,5-diamino-1-(2-pyrimidyl)benzene; 2,5-diamino-1-(6-methyl-2-pyridyl)-benzene; 2,5-diamino-1-(4-methyl-2-pyridyl)benzene; 2,5-diamino-1-(5-methyl-2-pyridyl)benzene and 2,5-diamino-1-(3-methyl-2-pyridyl)benzene or physiologically compatible salts thereof.

3. Composition for the oxidative colouring of keratin fibres, based on a developer substance-coupler substance combination, **characterized in that** it comprises at least one diaminobenzene derivative according to Claim 1 or 2 as developer substance.

4. Composition according to Claim 3, **characterized in that** the diaminobenzene derivative is present in an amount of from 0.005 to 20 per cent by weight.

5. Composition according to Claim 3 or 4, **characterized in that** the developer substances and coupler substances, based on the total amount of the oxidation colorant, are in each case present in a total amount of from 0.005 to 20 per cent by weight.

6. Composition according to one of Claims 3 to 5, **characterized in that** it additionally comprises at least one direct dye.

7. Composition according to one of Claims 3 to 6, **characterized in that** it has a pH of from 6.8 to 11.5.

8. Composition according to one of Claims 3 to 7, **characterized in that**, apart from the 1,4-diaminobenzene derivative according to Claim 1 or 2, it additionally comprises at least one further developer substance from the group consisting of 1,4-diaminobenzene, 2,5-diaminotoluene, 2,5-diaminophenylethanol, 4-aminophenol and its derivatives, 4,5-diaminopyrazole derivatives and tetraaminopyrimidines.

9. Composition according to one of Claims 3 to 8, **characterized in that** it is in the form of an aqueous or aqueous-alcoholic solution, a cream, a gel or an emulsion.

10. Composition according to one of Claims 3 to 9, **characterized in that** it is a hair colorant.

## Revendications

1. Dérivé de p-diaminobenzène, choisi parmi le 2,5-diamino-1-(2-pyridyl)benzène, le 2,5-diamino-1-(3-pyridyl)benzène, le 2,5-diamino-1-(4-pyridyl)benzène, le 2,5-diamino-4-méthoxy-1-(2-pyridyl)benzène, le 2,5-diamino-4-méthoxy-1-(3-pyridyl)benzène, le 2,5-diamino-4-méthoxy-1-(4-pyridyl)benzène, le 2,5-diamino-4-méthyl-1-(2-pyridyl)benzène, le 2,5-diamino-4-méthyl-1-(3-pyridyl)benzène, le 2,5-diamino-4-méthyl-1-(4-pyridyl)benzène, le 2,5-diamino-1-(3-amino-2-pyridyl)-benzène, le 2,5-diamino-1-(3-chloro-2-pyridyl)benzène, le 2,5-diamino-1-(3-fluoro-2-pyridyl)benzène, le 2,5-diamino-1-(3-hydroxy-2-pyridyl)benzène, le 2,5-diamino-1-(3-méthyl-2-pyridyl)benzène, le 2,5-diamino-1-(3-trifluorométhyl-2-pyridyl)benzène, le 2,5-diamino-1-(4-amino-2-pyridyl)benzène, le 2,5-diamino-1-(4-chloro-2-pyridyl)benzène, le 2,5-diamino-1-(4-fluoro-2-pyridyl)-benzène, le 2,5-diamino-1-(4-hydroxy-2-pyridyl)benzène, le 2,5-diamino-1-(4-méthyl-2-pyridyl)benzène, le 2,5-diamino-1-(4-trifluorométhyl-2-pyridyl)benzène, le 2,5-diamino-1-(5-amino-2-pyridyl)benzène, le 2,5-diamino-1-(5-chloro-2-pyridyl)benzène, le 2,5-diamino-1-(5-fluoro-2-pyridyl)benzène, le 2,5-diamino-1-(5-hydroxy-2-pyridyl)benzène, le 2,5-diamino-1-(5-méthyl-2-pyridyl)benzène, le 2,5-diamino-1-(5-trifluorométhyl-2-pyridyl)benzène, le 2,5-diamino-1-(6-amino-2-pyridyl)-benzène, le 2,5-diamino-1-(6-chloro-2-pyridyl)benzène, le 2,5-diamino-1-(6-fluoro-2-pyridyl)benzène, le 2,5-diamino-1-(6-hydroxy-2-pyridyl)benzène, le 2,5-diamino-1-(6-méthyl-2-pyridyl)benzène, le 2,5-diamino-1-(6-trifluorométhyl-2-pyridyl)benzène, le 2,5-diamino-1-(2-amino-3-pyridyl)benzène, le 2,5-diamino-1-(2-chloro-3-pyridyl)benzène, le 2,5-diamino-1-(2-fluoro-3-pyridyl)-benzène, le 2,5-diamino-1-(2-hydroxy-3-pyridyl)benzène, le 2,5-diamino-1-(2-méthyl-3-pyridyl)benzène, le 2,5-diamino-1-(2-trifluorométhyl-3-pyridyl)benzène, le 2,5-diamino-1-(4-amino-3-pyridyl)benzène, le 2,5-diamino-1-(4-chloro-3-pyridyl)benzène, le 2,5-diamino-1-(4-fluoro-3-pyridyl)benzène, le 2,5-diamino-1-(4-hydroxy-3-pyridyl)benzène, le 2,5-diamino-1-(4-méthyl-3-pyridyl)benzène, le 2,5-diamino-1-(4-trifluorométhyl-3-pyridyl)benzène, le 2,5-diamino-1-(5-amino-3-pyridyl)-benzène, le 2,5-diamino-1-(5-chloro-3-pyridyl)benzène, le 2,5-diamino-1-(5-fluoro-3-pyridyl)benzène, le 2,5-diamino-1-(5-hydroxy-3-pyridyl)benzène, le 2,5-diamino-1-(5-méthyl-3-pyridyl)benzène, le 2,5-diamino-1-(5-trifluorométhyl-3-pyridyl)benzène, le 2,5-diamino-1-(6-amino-3-pyridyl)benzène, le 2,5-diamino-1-(6-chloro-3-pyridyl)benzène, le 2,5-diamino-1-(6-fluoro-3-pyridyl)-benzène, le 2,5-diamino-1-(6-hydroxy-3-pyridyl)benzène, le 2,5-diamino-1-(6-méthyl-3-pyridyl)benzène, le 2,5-diamino-1-(6-trifluorométhyl-3-pyridyl)benzène, le 2,5-diamino-1-(2-amino-4-pyridyl)benzène, le 2,5-diamino-1-(2-chloro-4-pyridyl)benzène, le 2,5-diamino-1-(2-fluoro-4-pyridyl)benzène, le 2,5-diamino-1-(2-hydroxy-4-pyridyl)benzène, le 2,5-diamino-1-(2-méthyl-4-pyridyl)benzène, le 2,5-diamino-1-(2-trifluorométhyl-4-pyridyl)benzène, le 2,5-diamino-1-(3-amino-4-pyridyl)-benzène, le 2,5-diamino-1-(3-chloro-4-pyridyl)benzène, le 2,5-diamino-1-(3-fluoro-4-pyridyl)benzène, le 2,5-diamino-1-(3-hydroxy-4-pyridyl)benzène, le 2,5-diamino-l-(3-méthyl-4-pyridyl)benzène, le 2,5-diamino-1-(3-trifluorométhyl-4-pyridyl)benzène, le 2,5-diamino-1-(5-amino-4-pyridyl)benzène, le 2,5-diamino-1-(5-chloro-4-pyridyl)benzène, le 2,5-diamino-1-(5-fluoro-4-pyridyl)-benzène, le 2,5-diamino-1-(5-hydroxy-4-pyridyl)benzène, le 2,5-diamino-1-(5-méthyl-4-pyridyl)benzène, le 2,5-diamino-1-(5-trifluorométhyl-4-pyridyl)benzène, le 2,5-diamino-1-(6-amino-4-pyridyl)benzène, le 2,5-diamino-1-(6-chloro-4-pyridyl)benzène, le 2,5-diamino-1-(6-fluoro-4-pyridyl)benzène, le 2,5-diamino-1-(6-hydroxy-4-pyridyl)benzène, le 2,5-diamino-1-(6-méthyl-4-pyridyl)benzène, le 2,5-diamino-1-(6-trifluorométhyl-4-pyridyl)benzène, le 2,5-diamino-1-(2-pyrimidyl)benzène, le 2,5-diamino-1-(4-pyrimidyl)benzène, le 2,5-diamino-1-(5-pyrimidyl)benzène, le 2,5-diamino-1-(6-pyrimidyl)benzène, le 2-hydroxyéthylamino-5-amino-1-(2-pyridyl)benzène, le 2-hydroxyéthylamino-5-amino-1-(3-pyridyl)benzène, le 2-hydroxyéthylamino-5-amino-1-(4-pyridyl)benzène, le 2-bis(hydroxyéthyl)amino-5-amino-1-(2-pyridyl)benzène, le 2-bis(hydroxyéthyl)amino-5-amino-1-(3-pyridyl)benzène, le 2-bis(hydroxyéthyl)amino-5-amino-1-(4-pyridyl)benzène, le 2,5-diamino-1-(3-cyano-2-pyridyl)benzène, le 2,5-diamino-1-(3-nitro-2-pyridyl)benzène, le 2,5-diamino-1-(4-cyano-2-pyridyl)benzène, le 2,5-diamino-1-(4-nitro-2-pyridyl)benzène, le 2,5-diamino-1-(5-cyano-2-pyridyl)benzène, le 2,5-diamino-1-(5-nitro-2-pyridyl)benzène, le 2,5-diamino-1-(6-cyano-2-pyridyl)benzène, le 2,5-diamino-1-(6-nitro-2-pyridyl)-benzène, le 2,5-diamino-1-(2-cyano-4-pyridyl)benzène, le 2,5-diamino-1-(2-nitro-4-pyridyl)benzène, le 2,5-diamino-1-(3-cyano-4-pyridyl)benzène, le 2,5-diamino-1-(3-nitro-4-pyridyl)benzène, le 2,5-diamino-1-(5-cyano-4-pyridyl)benzène, le 2,5-diamino-1-(5-nitro-4-pyridyl)benzène, le 2,5-diamino-1-(6-cyano-4-pyridyl)-benzène, le 2,5-diamino-1-(6-nitro-4-pyridyl)benzène, le 2,5-diamino-1-(2-cyano-3-pyridyl)benzène, le 2,5-diamino-1-(2-nitro-3-pyridyl)benzène, le 2,5-diamino-1-(4-cyano-3-pyridyl)benzène, le 2,5-diamino-1-(4-nitro-3-pyridyl)benzène, le 2,5-diamino-1-(5-cyano-3-pyridyl)benzène, le 2,5-diamino-1-(5-nitro-3-pyridyl)-benzène, le 2,5-diamino-1-(6-cyano-3-pyridyl)benzène et le 2,5-diamino-1-(6-nitro-3-pyridyl)benzène ou leurs sels physiologiquement acceptables.

2. Dérivé de p-diaminobenzène selon la revendication 1, **caractérisé en ce qu'**il est choisi parmi le 2,5-diamino-1-(2-pyridyl)benzène, le 2,5-diamino-1-(3-pyridyl)benzène, le 2,5-diamino-1-(4-pyridyl)benzène, le 2,5-diamino-1-(2-pyrimidyl)benzène, le 2,5-diamino-1-(6-méthyl-2-pyridyl)benzène, le 2,5-diamino-1-(4-méthyl-2-pyridyl)benzène, le 2,5-diamino-1-(5-méthyl-2-pyridyl)benzène et le 2,5-diamino-1-(3-méthyl-2-pyridyl)benzène ou leurs sels physiologiquement acceptables.

3. Produit pour la teinture par oxydation de fibres kératiniques, à base d'une association de coupleur-développeur, **caractérisé en ce qu'**il contient comme développeur au moins un dérivé de diaminobenzène selon la revendication 1 ou 2.

4. Produit selon la revendication 3, **caractérisé en ce que** le dérivé de diaminobenzène est contenu en une quantité de 0,005 à 20 % en poids.

5. Produit selon la revendication 3 ou 4, **caractérisé en ce que** les coupleurs et les développeurs sont contenus chacun, par rapport à la quantité totale du produit de teinture par oxydation, en une quantité totale de 0,005 à 20 % en poids.

6. Produit selon l'une quelconque des revendications 3 à 5, **caractérisé en ce qu'**il contient en outre au moins un colorant direct.

7. Produit selon l'une quelconque des revendications 3 à 6, **caractérisé en ce qu'**il présente un pH de 6,8 à 11,5.

8. Produit selon l'une quelconque des revendications 3 à 7, **caractérisé en ce que**, outre le dérivé de 1,4-diaminobenzène selon la revendication 1 ou 2, il contient au moins un autre développeur choisi dans le groupe constitué par le 1,4-diaminobenzène, le 2,5-diaminotoluène, le 2,5-diaminophényléthanol, le 4-aminophénol et ses dérivés, des dérivés de 4,5-diaminopyrazole et des tétra-aminopyrimidines.

9. Produit selon l'une quelconque des revendications 3 à 8, **caractérisé en ce qu'**il se trouve sous forme d'une solution aqueuse ou aqueuse-alcoolique, d'une crème, d'un gel ou d'une émulsion.

10. Produit selon l'une quelconque des revendications 3 à 9, **caractérisé en ce qu'**il s'agit d'un produit de teinture pour cheveux.
